# EUROPÄISCHE PATENTANMELDUNG

(11) **EP 4 837 594 A1**
(43) Veröffentlichungstag der Anmeldung: **11.11.2026**
(21) Anmeldenummer: 25174289.6
(22) Anmeldetag: 05.05.2025
(51) Int. Cl.: A61L 2/00, A61L 2/14, A61L 2/18, A61L 2/24, A61L 2/28

(54) **REINIGUNGSANLAGE ZUR REINIGUNG VON MEDIZINISCHEN ODER PHARMAZEUTISCHEN INSTRUMENTEN ODER GÜTERN UND VERFAHREN ZUR REINIGUNG**

(71) Anmelder: Belimed Life Science AG, 8583 Sulgen (CH)
(72) Erfinder: Schlag, Jochen, 8280 Kreuzlingen (CH)
(74) Vertreter: Hepp Wenger Ryffel AG

(57) **Zusammenfassung**

Die Erfindung betrifft eine Reinigungsanlage (1), insbesondere Sterilisations- und/oder Desinfektionsanlagen, zur Reinigung von medizinischen oder pharmazeutischen Instrumenten oder Gütern. Die Reinigungsanlage umfasst einen Reinigungsraum (2) mit einer Reinigungseinrichtung (10), in dem die Güter reinigbar sind, einen Reinigungsfluidzulauf (3) zur Zuführung von Reinigungsfluid (100). Zudem ist ein Plasmareaktor (4) ausgebildet, durch den Wasser mit Plasma aktivierbar ist und Plasma im Plasmareaktor mittels Gas, vorzugsweise mittels Luft und/oder Stickstoff, herstellbar ist und das plasmaaktivierte Wasser als Reinigungsfluid (100) verwendbar ist. Die Erfindung betrifft zudem ein Verfahren zur Reinigung, insbesondere Desinfektion oder Sterilisierung von Gütern, insbesondere medizinischen und/oder pharmazeutischen Gütern, in einer Reinigungsanlage (1).

## Beschreibung

Die vorliegende Erfindung betrifft ein Reinigungsanlage, insbesondere Sterilisations- und/oder Desinfektionsanlage, zur Reinigung von medizinischen und/oder pharmazeutischen Instrumenten und/oder Gütern, sowie ein Verfahren zur Reinigung, insbesondere Desinfektion oder Sterilisierung von Gütern, insbesondere medizinischen und/oder pharmazeutischen Gütern, in einer Reinigungsanlage.

In verschiedenen Bereichen wie beispielsweise der Chemieindustrie, der Pharmazeutik und der Medizin sowie der Labortechnik ist das gründliche Reinigen von Materialien wie Ampullen etc. von grosser Wichtigkeit. Reinigungsanlagen zur Reinigung von medizinischen oder pharmazeutischen Instrumenten oder Güter sind aus dem Stand der Technik bekannt. Bei derartigen Reinigungsanlangen ist eine Keimreduktion auf und/oder in den Reinigungsgütern von grosser Wichtigkeit. Die keimreduzierende Wirkung wird im Stand der Technik durch unterschiedliche Chemikalien, insbesondere Desinfektionsmittel wie beispielsweise Alkohole erzielt. Derartige Reinigungsanlagen sind nachteilig, da diese Chemikalien nach ihrer Verwendung als Sonderabfall entsorgt, werden müssen. Weiterhin ist es bekannt, hohe Temperaturen zur Reinigung von Gütern oder Instrumenten zu nutzen. Dies führt zu hohem Energieverbrauch.

Es ist daher die Aufgabe der Erfindung die Nachteile des Standes der Technik zu überwinden und insbesondere eine Reinigungsanlange sowie ein Verfahren zur Reinigung, insbesondere Desinfektion oder Sterilisierung, von medizinischen und/oder pharmazeutischen Instrumenten und/oder Gütern zu schaffen, durch welches die Effizienz und Nachhaltigkeit der Reinigung optimiert wird.

Die Aufgabe wird durch ein Reinigungsanlage, insbesondere Sterilisations- und/oder Desinfektionsanlage, zur Reinigung von medizinischen und/oder pharmazeutischen Instrumenten und/oder Gütern, sowie durch ein Verfahren zur Reinigung, insbesondere Desinfektion und/oder Sterilisierung von Gütern, insbesondere medizinischen und/oder pharmazeutischen Gütern, gemäss den unabhängigen Ansprüchen gelöst.

Insbesondere wird die Aufgabe durch eine Reinigungsanlage, insbesondere Sterilisations- und/oder Desinfektionsanlage, zur Reinigung von medizinischen oder pharmazeutischen Instrumenten oder Gütern gelöst. Die Reinigungsanlage umfasst einen Reinigungsraum mit einer Reinigungseinrichtung, in dem die Güter reinigbar sind und einen Reinigungsfluidzulauf zur Zuführung von Reinigungsfluid. Zudem ist ein Plasmareaktor ausgebildet, durch den Wasser mit Plasma aktivierbar ist und Plasma im Plasmareaktor mittels Gas, vorzugsweise mittels Luft und/oder Stickstoff, herstellbar ist und das plasmaaktivierte Wasser als Reinigungsfluid verwendbar ist.

Eine Reinigungsanlage kann Teil eines, insbesondere automatisierten, Reinigungssystems sein, welches insbesondere für hohe Durchsätze von Reinigungsgütern vorteilhaft ist.

Der Reinigungsraum kann einen oder mehrere Beladungsträger aufnehmen. Reinigungsgüter können auf den Beladungsträgern angeordnet sein, so dass eine Reinigung und/oder Desinfektion und/oder Sterilisations durchführbar ist. Reinigungsgüter können medizinischen und/oder pharmazeutischen Instrumenten und/oder Güter umfassen. Der Reinigungsraum kann auch derart ausgebildet sein, dass Reinigungsgüter ohne Beladungsträger reinigbar und/oder desinfizierbar sind.

Der Reinigungsraum kann über eine Beladungsöffnung öffenbar sein. Die Be- und/oder Entladung des Reinigungsraums mit Reinigungsgütern kann automatisiert und/oder manuell erfolgen. Eine automatisierte Be- und/oder Entladung ist insbesondere für hohe Durchsätze an Reinigungsgütern vorteilhaft. Eine manuelle Be- und/oder Entladung ist für kleine Reinigungsräume und/oder kleine Durchsätze an Reinigungsgütern vorteilhaft.

Eine Reinigungseinrichtung bezeichnet eine Einrichtung, durch welche die Reinigungs- und/oder Desinfektions- und/oder Sterilisationsleistung der Reinigungsanlage verbesserbar ist. Insbesondere kann durch eine Reinigungseinrichtung Reinigungsfluid im Reinigungsraum und/oder an den Reinigungsgütern verteilbar sein. Durch eine Reinigungseinrichtung können Reinigungsgüter im Reinigungsraum bewegbar sein.

Durch eine Reinigungsfluidzulauf kann ein oder mehrere Reinigungsfluide dem Reinigungsraum zuführbar sein. Der Reinigungsfluidzulauf kann eine starre und/oder flexible Leitung umfassen.

Die Reinigungseinrichtung kann zwei oder mehrere Reinigungsfluidzuläufe umfassen. Durch eine derartige Ausführungsform kann eine gleichmässige Verteilung eines oder mehrerer Reinigungsfluide im Reinigungsraum erreichbar sein. Des Weiteren kann bei der Verwendung unterschiedliche Reinigungsfluide jeweils ein Reinigungsfluidzulauf für die Zugabe eines Reinigungsfluids verwendbar sein.

Das Reinigungsfluid kann plasmaaktiviertes Wasser umfassen. Das plasmaaktiviertes Wasser kann vorzugsweise gereinigtes Wasser oder Reinstwasser umfassen. In einer Ausführungsform kann das Reinigungsfluid mit Zusätzen versetzt oder versetzbar sein.

Plasmaaktiviertes Wasser ist besonders vorteilhaft, da durch die Behandlung, insbesondere durch Mischen, von Wasser mit Plasma reaktive Spezies, insbesondere reaktive Sauerstoffspezies, bildbar sind. Insbesondere reaktive Sauerstoffspezies wie Wasserstoffperoxid und/oder Ozon sind durch ihre keimreduzienrende Wirkung besonders vorteilhaft für die Reinigung und/oder Desinfektion von Reinigungsgütern und/oder der Reinigungsanlage selbst. Plasmaaktiviertes Wasser kann sanitisierend und/oder desinfizierend und/oder sterilisierend wirken. Plasmaaktiviertes Wasser kann zudem rückstandslos von Reinigungsgütern entfernbar sein.

Die Reinigung und/oder Desinfektion von Reinigungsgütern mittels plasmaaktiviertem Wasser als Reinigungsfluid ist zudem vorteilhaft, da nach der Reinigung und/oder Desinfektion das Reinigungsfluid nicht mittels Sonderabfall entsorgt werden muss. Die reaktiven Spezies besitzen kurze Halbwertszeiten, weshalb die keimreduzierende Wirkung des plasmaaktivierten Wassers über die Zeit nachlässt. Eine gezielte Inaktivierung der reaktiven Spezies oder des plasmaaktivierten Wassers ist deshalb nicht notwendig. Durch Reinigungsgüter verunreinigtes Reinigungsfluid kann demnach in einer Abwasserreinigungsanlage aufbereitet werden.

Mit einem Plasmareaktor kann Plasma herstellbar sein. Mittels Plasmareaktor kann vorzugsweise Atmosphärendruckplasma, auch kaltes Plasma genannt, herstellbar sein. Derartiges Plasma ist besonders vorteilhaft für die Herstellung von plasmaaktiviertem Wasser, da die Herstellung sicher und energieeffizient ist. Zudem können unerwünschte thermische Effekte vermieden werden.

Das Plasma ist vorzugsweise mittels Gas und/oder Gasgemisch herstellbar. In einer bevorzugten Ausführungsform kann das Plasma mittels Luft herstellbar sein. Luft ist für die Herstellung von Plasma besonders geeignet, da die Herstellung kostengünstig erfolgen kann.

Der Plasmareaktor kann einen Hochspannungsgenerator und eine Plasmadüse umfassen. Der Plasmareaktor kann einen oder mehrere Gaseingänge umfassen, durch welche Gas, insbesondere komprimiertes Gas, in den Plasmareaktor gelangen kann. Das Gas, insbesondere komprimierte Luft, kann durch die Plasmadüse geleitet werden, welche zwei Elektroden umfasst. Das Gas kann die Elektroden umströmen. Durch eine Hochspannungsentladung zwischen den Elektroden kann aus dem Gas Plasma bildbar sein. Der Plasmareaktor kann einen oder mehrere Kühlkörper umfassen, wodurch das Plasma kühlbar ist. Das Plasma kann über einen Plasmaausgang aus dem Plasmareaktor leitbar sein. Das erzeugte Plasma kann mit Wasser gemischt werden, wodurch plasmaaktiviertes Wasser bildbar ist, welches als Reinigungsfluid verwendbar ist.

Die keimreduzierende Wirkung des plasmaaktivierten Wassers kann durch die elektrische Spannung und/oder die elektrische Stromstärke an den Elektroden des Plasmareaktors, und/oder den Volumenstrom des Gases und/oder des Gasgemisches, und/oder der Verweilzeit des Plasmas im Reinigungsfluid beinflussbar sein.

Die Reinigungseinrichtung kann zumindest einen beweglichen Wascharm umfassen.

Eine Reinigungseinrichtung, welche einen Wascharm umfasst, ist vorteilhaft, da durch den Wascharm ein Reinigungsfluid kostengünstig und effizient im Reinigungsraum verteilbar ist. Dadurch können Reinigungsgüter im Reinigungsraum effizient gereinigt und/oder desinfiziert werden.

Ein beweglicher Wascharm kann im Reinigungsraum derart angeordnet sein, dass der Wascharm bei einem mit einem Beladungsträger belandere Reinigungsraum unterhalb oder oberhalb eines Beladungsträger angeordnet ist. Der Wascharm kann auch zwischen zwei Beladungsträgern angeordnet sein. Die Reinigungseinrichtung kann zwei oder mehr bewegliche Wascharme umfassen. Eine derartige Ausführungsform kann die Reinigungsfluidverteilung und/oder die Reinigungsleistung verbessern.

In einer bevorzugten Ausführungsform kann die Reinigungseinrichtung zwei bewegliche Wascharme umfassen, wobei ein Wascharm unter einem Beladungsträger und ein Wascharm oberhalb des Beladungsträger angeordnet ist.

Der bewegliche Wascharm kann mechanisch antreibbar sein und/oder hydraulisch, durch ein durchströmendes Reinigungsfluids, antreibbar sein.

Die Reinigungseinrichtung kann ein Reinigungsbecken und eine Reinigungstrommel und vorzugsweise mindestens eine Sprühdüse umfassen, wobei die Reinigungstrommel zumindest teilweise im Reinigungsbecken angeordnet sein kann.

Das Reinigungsbecken kann dieselben Abmasse aufweisen wie der Reinigungsraum, und zumindest teilweise mit einem Reinigungsfluid befüllt oder befüllbar sein. Insbesondere kann das Reinigungsbecken derart mit Reinigungsfluid befüllt sein, dass die Reinigungstrommel zumindest teilweise vom Reinigungsfluid des Reinigungsbeckens benetzbar ist.

Die Reinigungsgüter können lösbar an einer Reinigungstrommel angeordnet sein. Durch eine Rotation der Reinigungstrommel kann eine gleichmässige Reinigung und/oder Desinfektion der Reinigungsgüter erfolgen.

Eine derartige Reinigungseinrichtung kann mindestens eine Sprühdüse umfassen, welche vorzugsweise oberhalb der Reinigungstrommel angeordnet ist. Durch die mindestens eine Sprühdüse kann Reinigungsfluid in den Reinigungsraum, insbesondere auf die Reinigungsgütern aufgebracht werden. Durch eine derartige Ausführungsform kann die Reinigungsleistung verbessert werden.

Die Reinigungseinrichtung kann ein Reinigungsbecken und einen Ultraschallgenerator umfassen.

Die Reinigungseinrichtung kann ein, wie vorgängig beschriebenes, Reinigungsbecken umfassen. In einer Ausführungsform können ein oder mehrere Beladungsträger im Reinigungsbecken angeordnet sein. Das Reinigungsbecken kann derart mit Reinigungsfluid befüllt sein, dass die Beladungsträger zumindest teilweise, vorzugsweise vollständig, mit Reinigungsfluid bedeckt sind. Durch eine derartige Ausführungsform können die Reinigungsgüter möglichst vollständig mit Reinigungsfluid benetzbar sein.

Die Reinigungseinrichtung kann mindestens einen Ultraschallgenerator umfassen, welcher am und/oder im Reinigungsbecken angeordnet ist. Durch den Ultraschallgenerator können Ultraschallwellen erzeugbar sein, durch welche die Reinigungsleistung und/oder Desinfektionsleitung verbessert werden kann.

Die Reinigungsanlage kann eine Plasmaleitung und eine Plasmaverteilungsvorrichtung, insbesondere eine Plasmadispergiervorrichtung, umfassen.

Durch eine Plasmaleitung kann das im Plasmareaktor erzeugte Plasma zum Reinigungsfluid leitbar sein. Das Plasma kann in der Plasmaleitung einen Druck von 1 bar - 8 bar aufweise. Dadurch ist verhinderbar, dass Wasser oder anders Reinigungsfluid in die Plasmaleitung und/oder den Plasmareaktor gelangt.

Die Plasmaleitung kann eine Absperrarmatur, insbesondere ein Rückschlagventil umfassen. Dadurch kann verhindert werden, dass Reinigungsfluid in den Plasmareaktor gelangen kann. Die Plasmaleitung kann ein steuerbares Ventil umfassen, durch welches die Plasmaleitung öffenbar und/oder schliessbar ist. Ein derartiges Ventil kann abhängig von einem Reinigungsschritt öffenbar und/oder schliessbar sein. Die Plasmaleitung kann flexibel oder starr ausgebildet sein. Eine flexible Leitung, insbesondere ein Schlauch, ist vorteilhaft, da dadurch die Plasmaleitung flexibel anordenbar ist. Eine starre Plasmaleitung ist vorteilhaft, da diese robust und druckbeständiger ausbildbar ist. Zudem ist das Hygienedesign einer starren Plasmaleitung vorteilhaft gegenüber einer flexiblen Plasmaleitung.

Eine Plasmaverteilvorrichtung kann ein verteilen des Plasmas in einem Reinigungsfluid, insbesondere Wasser, ermöglichen und/oder verbessern. Die Plasmaverteilvorrichtung ist vorzugsweise als Plasmadispergiervorrichtung ausgebildet, welche ein Dispergieren von Plasmablasen im Reinigungsfluid ermöglicht.

Fein dispergiertes Plasma, insbesondere Plasmablasen mit einer hohen volumenbezogenen spezifischen Oberfläche sind vorteilhaft, da dadurch das Reinigungsfluid schneller und effizienter plasmaaktiverbar ist.

Die Plasmadispergiervorrichtung kann als Sparger, insbesondere als Mikrosparger ausgebildet sein. Ein Sparger kann als Lochplatte ausgebildet sein. Ein derartiger Sparger ist einfach herstellbar und robust. Ein Mikrosparger kann ein gesintertes Element umfassen. Das Plasma kann durch das gesinterte Element gleitet werden, wodurch Plasmablasen mit einer grösseren volumenbezogenen spezifischen Oberfläche erzeugbar sind als bei der Verwendung einer Lochplatte. Die Reinigungsleistung und/oder Desinfektionsleistung, insbesondere die keimreduzierende Wirkung, kann durch verbesserbar sein.

Bevorzugt weist das Reinigungsfluid ein turbulentes Strömungsverhalten auf, wodurch die Verweilzeit der Plasmablasen verbesserbar ist. Dadurch ist die Reinigungsleistung und/oder Desinfektionsleistung, insbesondere die keimreduzierende Wirkung, verbesserbar.

In einer bevorzugten Ausführungsform kann das Plasma mittels Plasmadispergiervorrichtung in eine Reinigungsfluidleitung, insbesondere eine Umwälzleitung, geleitet werden, in welcher das Reinigungsfluid turbulentes Strömungsverhalten aufweist. In einer derartigen Ausführungsform kann eine besonders hohe Reinigungsleistung und/oder Desinfektionsleistung, insbesondere keimreduzierende Wirkung, erreichbar sein.

Der Plasmareaktor kann über die Plasmaleitung direkt oder indirekt mit dem Reinigungsraum verbunden sein, insbesondere mit dem Sumpf eines Reinigungsraumes.

Dadurch eine direkte Verbindung des Plasmareaktors über die Plasmaleitung mit dem Reinigungsraum kann das Plasma direkt in den Reinigungsraum einleitbar sein. Eine derartige Plasmaleitung ist vorzugsweise so angeordnet, dass das Plasma in das Reinigungsfluid leitbar ist. Durch eine derartige Ausführungsform kann eine kompakte und robuste Reinigungsanlage bereitgestellt werden.

Der Reinigungsraum kann an dessen tiefsten Stelle einen Sumpf umfassen, in welchem Reinigungsfluid sammelbar ist. Die Plasmaleitung kann derart ausgebildet sein, dass das Plasma in den Sumpf des Reinigungsraums leitbar ist. Eine derartige Ausführungsform ist besonders einfach und kompakt bereitstellbar.

Dadurch eine indirekte Verbindung des Plasmareaktors über die Plasmaleitung mit dem Reinigungsraum kann das Plasma in einen Behälter und/oder eine Leitung, insbesondere in einen Umlaufleitung, einleitbar sein. Durch eine derartige Ausführungsform kann die Verweilzeit des Plasmas im Reinigungsfluid verbessert werden, wodurch die Reinigungsleistung und/oder Desinfektionsleistung, insbesondere die keimreduzierende Wirkung, verbesserbar ist.

In einer bevorzugten Ausführungsform umfasst der Reinigungsraum einen Sumpf, in welchem Reinigungsfluid sammelbar ist. Das Reinigungsfluid kann durch eine Umwälzpumpe und eine Umwälzleitung in mindestens einen Reinigungsfluidzulauf pumpbar sein. Das Reinigungsfluid kann über den mindesten einen Reinigungsfluidzulauf zurück in den Reinigungsraum beförderbar sein. Plasma kann mittels Plasmaverteilungsvorrichtung in die Umwälzleitung einleitbar sein. Die Umwälzpumpe und/oder die Umwälzleitung können derart ausgebildet sein, dass das Reinigungsfluid in der Umwälzleitung turbulentes strömungsverhalten aufweist. Eine derartige Ausführungsform besonders vorteilhaft, da eine hohe Reinigungsleistung, insbesondere die keimreduzierende Wirkung, erreichbar ist.

Es kann eine Aufbereitungsanlage ausgebildet sein, in der das Reinigungsfluid aufbereitbar ist.

In einer Aufbereitungsanlage kann das Reinigungsfluid direkt oder indirekt aufbereitbar, insbesondere mit Plasma mischbar sein.

Die keimreduzierende Wirkung des plasmaaktivierten Wassers kann mit der Zeit, insbesondere durch Reduktion der reaktiven Spezies, abnehmen. Eine Aufbereitung des Reinigungsfluids in einer Aufbereitungsanlage kann deshalb vorteilhaft sein, um eine gleichbleibende keimreduzierende Wirkung des Reinigungsfluids aufrechtzuerhalten.

Die Aufbereitungsanlange kann als Tank mit mindestens einem Reinigungsfluidzulauf und mindestes einem Reinigungsfluidablauf ausgebildet sein. Die Aufbereitungsanlange kann vorzugsweise eine Abluftöffnung umfassen, durch welche Abluft aus der Aufbereitungsanlange abführbar ist.

Der Reinigungsfluidzulauf ist vorzugsweise im Kopfbereich der Aufbereitungsanlange angeordnet und der Reinigungsfluidablauf am tiefsten Punkt der Aufbereitungsanlange. Dadurch kann eine Kurzschlussströmung verhinderbar sein und die Aufbereitungsanlange kann selbstentleerend ausgebildet sein.

Der Reinigungsfluidablauf der Aufbereitungsanlange kann direkt oder indirekt mit einem oder mehreren Reinigungsfluidzuläufen verbunden sein. Dadurch kann das aufbereitet Reinigungsfluid erneut zur Reinigung und/oder Desinfektion im Reinigungsraum verwendet werden.

In einer bevorzugten Ausführungsform ist der Reinigungsfluidzulauf und/oder eine Verbindungsleitung, welche den Reinigungsfluidablauf der Aufbereitungsanlange mit dem Reinigungsfluidzulauf verbindet, möglichst kurz ausgebildet. Dies ist vorteilhaft, da dadurch das aufbereitet Reinigungsfluid schnell und möglichst ohne Reduktion der keimreduzierenden Wirkung in den Reinigungsraum, insbesondere auf die Reinigungsgüter leitbar ist.

Die Plasmaleitung kann vom Plasmareaktor zur Aufbereitungsanlage ausgebildet sein und der Reinigungsfluidzulauf kann von der Aufbereitungsanlage zum Reinigungsraum verlaufen.

Die Plasmaleitung kann wie vorgängig beschrieben ausgebildet sein. Das Plasma kann im unteren Bereich der Aufbereitungsanlage einbringbar sein, so dass das Plasma durch das in der Aufbereitungsanlage befindliche Wasser aufsteigbar ist. Vorzugsweise ist im unteren Bereich der Aufbereitungsanlage, insbesondere am Boden der Aufbereitungsanlage, eine Plasmaverteilungsvorrichtung, vorzugsweise wie vorgängig beschrieben, ausgebildet.

Die Aufbereitungsanlage kann zylinderförmig ausgebildet sein und vorzugsweise eine Höhe umfassen, welche grösser als der Querschnitt, insbesondere der Durchmesser, der Aufbereitungsanlage ausgebildet ist. Insbesondere kann die Höhe der Aufbereitungsanlange 1.5- bis 5-mal grösser als der Querschnitt der Aufbereitungsanlage ausgebildet sein. Durch ein derartige Aufbereitungsanlage kann die Verweilzeit des Plasmas im Reinigungsfluid verlängert werden. Durch die längere Verweilzeit kann die keimreduzierende Wirkung des Reinigungsfluids, insbesondere des plasmaaktivierten Wassers, verbessert werden.

In einer bevorzugten Ausführungsform ist die Plasmaverteilungsvorrichtung als Mikrosparger mit einem gesinterten Element ausgebildet. Die Plasmaverteilungsvorrichtung kann einen Querschnitt aufweisen, welcher mindestens 10%, insbesondere mindestens 30% des Querschnitts der Aufbereitungsanlage entspricht. Dadurch kann eine gleichmässige Verteilung des Plasmas in der Aufbereitungsanlange errichbar sein. In einer bevorzugten Ausführungsform entspricht der Querschnitt der Plasmaverteilungsvorrichtung im Wesentlichen dem Querschnitt der Aufbereitungsanlage. Dadurch kann eine besonders gleichmässige Verteilung des Plasmas in der Aufbereitungsanlange errichbar sein.

Die Aufbereitungsanlange kann einen Rührer umfassen. Dadurch kann eine turbulente Strömung in der Aufbereitungsanlange erreichbar sein und/oder Plasmablasen können dispergierbar sein. Dadurch kann die Aufbereitung des Reinigungsfluids mit Plasma verbessert werden, wodurch die keimreduzierende Wirkung verbesserbar ist. Des Weiteren kann stehendes Reinigungsfluid vermieden werden.

Die Aufbereitungsanlage kann eine Umlaufleitung mit einer Umlaufpumpe umfassen.

Durch die Umlaufleitung kann Reinigungsfluid von Reinigungsfluidablauf der Aufbereitungsanlage zum Reinigungsfluidzulauf mittels Umlaufpumpe förderbar sein. Durch eine derartige Ausführungsform kann stehendes Reinigungsfluid, und dadurch eine Biofilmbildung vermeidbar sein. Des Weiteren kann durch das Umwälzen die Mischung des Reinigungsfluids mit Plasma verbessert werden.

Es kann eine Qualitätsüberwachungsvorrichtung zur Überwachung des plasmaktivierten Wassers ausgebildet sein.

Wie vorgängig beschrieben kann die keimreduzierende Wirkung des Reinigungsfluids, insbesondere des plasmaaktivierten Wassers über die Zeit, durch Reduktion, abnehmen. Durch eine Qualitätsüberwachungsvorrichtung kann während eines oder mehreren Reinigungs- und/oder Desinfektionsverfahrens eine konstante keimreduzierende Wirkung des Reinigungsfluids, insbesondere des plasmaaktivierten Wassers, gewährleistbar sein.

Bei einer detektierten Veränderung der keimreduzierenden Wirkung durch die Qualitätsüberwachungsvorrichtung kann das Reinigungsfluid verworfen und/oder aufbereitet werden und/oder zusätzliches Plasma kann dem Reinigungsfluid zugeführt werden.

Die Qualitätsüberwachungsvorrichtung kann im Reinigungsraum, insbesondere im Sumpf des Reinigungsraums angeordnet sein. Eine derartige Ausführungsform ist vorteilhaft, da die Qualität direkt im Reinigungsraum messbar ist. Die Qualitätsüberwachungsvorrichtung kann in der Aufbereitungsanlage angeordnet sein. Dadurch kann sichergestellt werden, dass das aufbereitete Reinigungsfluid die geforderte Qualität, insbesondere keimreduzierende Wirkung, aufweist, bevor dieses in den Reinigungsraum geleitet wird. Die Qualitätsüberwachungsvorrichtung kann in einer oder mehreren Flüssigkeitszuläufen und/oder Umwälzleitungen angeordnet sein. Durch eine derartige Ausführungsform kann die Qualität des Reinigungsfluids vor dem Einleiten in den Reinigungsraum bestimmbar sein.

Die Reinigungsanlage kann eine oder mehrere Qualitätsüberwachungsvorrichtungen umfassen, wobei die einzelnen Qualitätsüberwachungsvorrichtungen wie vorgängig beschrieben angeordnet sein können.

Die Qualitätsüberwachungsvorrichtung kann einen PH-Sensor und/oder einen Leitfähigkeitssensor und/oder einen Temperatursensor und/oder einen ORP (oxidation-reduction-potential)-Sensor und/oder einen TOC (total-organic-carbon)-Sensor umfassen.

Plasmaaktiviertes Wasser, kann durch die reaktive Spezies sauer sein. Da durch eine Abnahme der reaktiven Spezies im plasmaaktivierten Wasser die keimreduzierende Wirkung verringert wird und der pH-Wert erhöht wird, kann die Qualität des plasmaaktivierten Wassers mittels pH-Sensor überwachbar sein. Eine Qualitätsüberwachungsvorrichtung, welche einen PH-Sensor umfasst, ist besonders vorteilhaft, da pH-Sensoren kostengünstig sind und eine kurze Ansprechzeit aufweisen.

Durch einen ORP-Sensor, auch Redoxpotential-Sensor, kann das Redoxpotential des plasmaaktivierten Wassers messbar sein. Da durch eine Abnahme der reaktiven Spezies im plasmaaktivierten Wasser die keimreduzierende Wirkung verringert wird und das Redoxpotential verringert wird, kann die Qualität des plasmaaktivierten Wassers mittels ORP-Sensors überwachbar sein. Eine Qualitätsüberwachungsvorrichtung, welche einen ORP-Sensor umfasst, ist besonders vorteilhaft, da dadurch schnell und zuverlässig die keimreduzierende Wirkung indirekt messbar ist.

Eine Qualitätsüberwachungsvorrichtung, welche einen TOC-Sensor umfasst, ist vorteilhaft, da dadurch organische Verunreinigungen im Reinigungsfluid messbar sind. Eine Qualitätsüberwachungsvorrichtung, welche einen Leitfähigkeitssensor umfasst, ist vorteilhaft, da dadurch Verunreinigungen im Reinigungsfluid messbar sind. Eine Qualitätsüberwachungsvorrichtung, welche einen Temperatursensor umfasst, ist vorteilhaft, da dadurch die Temperatur des Reinigungsfluids messbar ist.

Eine Qualitätsüberwachungsvorrichtung kann redundant, insbesondere doppelt, ausgebildet sein, wodurch die Prozesssicherheit verbesserbar ist.

Die Messwerte der Qualitätsüberwachungsvorrichtung können zur Regelung des Plasmareaktors und/oder einer oder mehrere Umwälzpumpen und/oder zur Steuerung von Ventilen und/oder anderen Armaturen verwendbar sein. Durch die Regelung des Plasmareaktors kann die produzierbare Plasmamenge, insbesondere der Volumenstrom, und/oder der Plasmaparameter anpassbar sein. Durch die Regelung einer oder mehrere Umwälzpumpen kann Reinigungsfluid in die Aufbereitungsanlage pumpbar sein und/oder verworfen werden.

Die Aufgabe wird weiter durch ein Verfahren zur Reinigung, insbesondere Desinfektion oder Sterilisierung von Gütern, insbesondere medizinischen und/oder pharmazeutischen Gütern, in einer Reinigungsanlage gelöst. Zunächst wird aus Wasser mit Plasma durch Vermischen plasmaaktiviertes Wasser erstellt. Das plasmaaktivierte Wasser, insbesondere das plasmaaktive Wasser und ein zusätzliches Reinigungsmittel, wird zum Reinigen von Gütern in einem Reinigungsraum verwendet.

Gas, insbesondere Luft und/oder Stickstoff, kann hierzu in einen, vorzugsweise wie vorgängig beschriebenen, Plasmareaktor geleitet werden. Durch eine Hochspannungsentladung kann Plasma herstellbar sein. Das Plasma kann mit Wasser oder mit einem Reinigungsfluid gemischt werden. Das Plasma kann hierzu, vorzugsweise über eine Plasmaverteilungsvorrichtung, in das Reinigungsfluid eingeleitet werden. Das Reinigungsfluid, welches plasmaaktivertes Wasser umfasst, kann direkt oder indirekt in den Reinigungsraum geleitet werden und dadurch, die dort angeordneten Reinigungsgüter reinigen und/oder desinfizieren. Im Reinigungsraum können die Reinigungsgüter auf einem oder mehreren Beladungsträgern oder einer Reinigungstrommel angeordnet sein. Das Reiniungsfluid kann im Sumpf des Reinigungsraum sammelbar sein und durch eine Umwälzpumpe, eine Umwälzleitung und einen Reinigungsfluidzulauf erneut dem Reinigungsraum zugeführt werden oder verworfen werden.

Das plasmaaktivierte Wasser kann in einer Aufbereitungsanlage gesammelt werden.

Das Reinigungsfluid, insbesondere das plasmaaktivierte Wasser, kann vom Sumpf des Reinigungsraums in die Aufbereitungsanlage, welche vorzugsweise wie vorgängig beschrieben ausgebildet ist, mittels Umwälzpumpe förderbar sein. Dadurch kann das plasmaaktivierte Wasser erneut mit Plasma vermischt werden, wodurch die keimreduzierende Wirkung verbessert und/oder aufrechterhalten bleiben kann. In der Aufbereitungsanlage kann das Reinigungsfluid, insbesondere das plasmaaktivierte Wasser, weiter aufbereitet werden.

Das plasmaaktivierte Wasser kann in den Reinigungsraum geleitet werden.

Das Reinigungsfluid, insbesondere das plasmaaktivierte Wasser, kann anschliessend erneut dem Reinigungsraum zuführbar sein. Zudem kann frisches Reinigungsfluid, insbesondere Wasser, über eine Zuleitung in die Aufbereitungsanlage leitbar sein. Derartiges Reinigungsfluid kann erstmalig dem Reinigungsraum zuführbar sein.

Das Reinigungsfluid, welches in den Reinigungsraum geleitet wird, kann in Kontakt mit den Reinigungsgütern kommen, wodurch diese gereinigt und/oder desinfiziert werden können. Das Reinigungsfluid, welches in den Reinigungsraum geleitet wird, kann in Kontakt mit den Reinigungsraum selbst kommen, wodurch dieser gereinigt und/oder desinfiziert werden kann.

Das plasmaaktivierte Wasser kann nach der Herstellung innerhalb von maximal 2 Stunden, insbesondere 1 Stunde, zum Reinigen im Reinigungsraum verwendet werden.

Die keimreduzierende Wirkung des Reinigungsfluids, insbesondere des plasmaaktivierten Wassers, kann reduziert werden, sobald das Reinigungsfluid nicht mehr mit Plasma, gemischt wird und/oder mit diesem in Kontakt gebracht wird. Deshalb ist eine rasche Verwendung vorteilhaft.

Die Verwendung des Reinigungsfluids innerhalb von maximal 2 Stunden, insbesondere 1 Stunde ist besonders vorteilhaft, da in dieser Zeit die keimreduzierende Wirkung des Reinigungsfluids zur Reinigung und/oder Desinfektion aufrechterhalten bleiben kann. Die Zeit zwischen Herstellung und Verwendung kann durch kurze Fliesswege des Reinigungsfluids reduzierbar sein. Die Zeit kann auch dadurch reduziert werden, dass Reinigungsfluid umgewälzt werden kann und dadurch kontinuierlich mit Plasma in Kontakt kommen kann.

Das plasmaaktivierte Wasser kann mit einer Qualitätsüberwachung überwacht werden.

Die Qualitätsüberwachung kann mit einer Quältitätsüberwachungsvorrichtung wie vorgängig beschrieben erfolgen. Dadurch kann eine konstante keimreduzierende Wirkung erreichbar sein und Verunreinigungen im Reinigungsfluid können frühzeitig erkennbar sein.

Die Erfindung wird in den folgenden Figuren näher erläutert. Es zeigen:
- **FIG 1**:: Schematische Darstellung einer Reinigungsanlage mit Zugabe des plasmaaktivierten Wassers in der Zirkulationsleitung,
- **FIG 2:**: Schematische Darstellung einer Reinigungsanlage mit Zugabe des plasmaaktivierten Wassers im Sumpf des Reinigungsraumes,
- **FIG 3:**: Schematische Darstellung einer Reinigungsanlage mit Zugabe des plasmaaktivierten Wassers in der Zirkulationsleitung der Aufbereitungsanlage,
- **FIG 4:**: Schematische Darstellung einer Reinigungsanlage mit Zugabe des plasmaaktivierten Wassers in der Aufbereitungsanlage,
- **FIG 5:**: Schematische Darstellung einer Reinigungsanlage mit Zugabe des plasmaaktivierten Wassers in der Zirkulationsleitung und einer Qualitätsüberwachungsvorrichtung im Reinigungsraum,
- **FIG 6:**: Schematische Darstellung einer Reinigungsanlage mit Zugabe des plasmaaktivierten Wassers in der Aufbereitungsanlag einer Qualitätsüberwachungsvorrichtung in der Aufbereitungsanlage,
- **FIG 7:**: Schematische Darstellung einer Reinigungsanlage mit einer Reinigungstrommel,
- **FIG 8:**: Schematische Darstellung einer Reinigungsanlage mit einem Ultraschallgenerator,
- **FIG 9:**: Schematische Darstellung einer Reinigungsanlage mit einem Ultraschallgenerator.

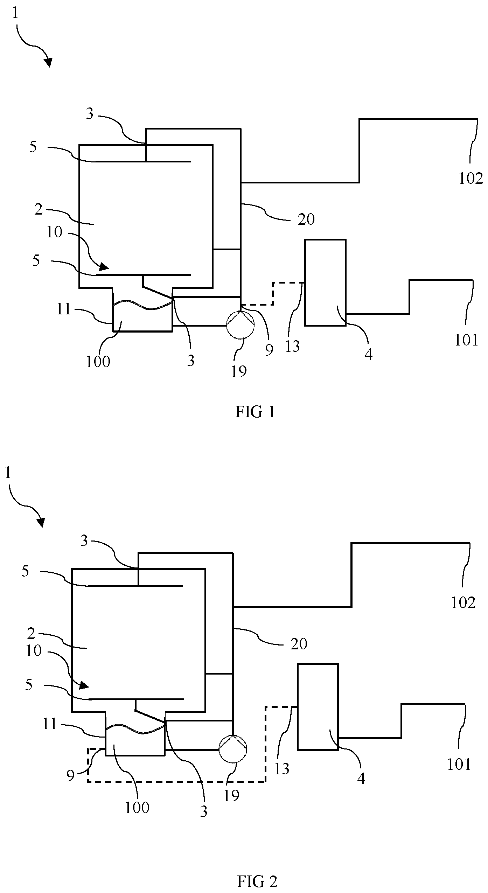

**Figur 1** zeigt eine schematische Darstellung einer Reinigungsanlage 1 mit Zugabe des plasmaaktivierten Wassers in der Zirkulationsleitung 20.

Die Reinigungsanlage 1 umfasst einen Reinigungsraum 2, welcher eine Reinigungseinrichtung 10 und einen Sumpf 11 umfasst. Die Reinigungseinrichtung 10 umfasst zwei bewegliche Wascharme 5, wobei ein Wascharm 5 im oberen Bereich des Reinigungsraums 2 und ein Wascharm 5 im unteren Bereich des Reinigungsraums 2 angeordnet ist. Dadurch können Reinigungsgüter, welche zwischen den zwei Wascharmen 5 anordenbar sind, effizient reinigbar und/oder desinfizierbar sein. Im Sumpf 11 ist das Reinigungsfluid 100 sammelbar.

Die Reinigungsanlange 1 umfasst weiter eine Umwälzpumpe 19, eine Umwälzleitung 20, zwei Reinigungsfluidzuläufe 3 und einen Reinigungsfluideingang 102. Ein Reinigungsfluidzulauf 3 ist im oberen Bereich des Reinigungsraums 2 angeordnet, wodurch der obere Wascharm 5 mit Reinigungsfluid 100 speisbar ist. Ein Reinigungsfluidzulauf 3 ist im unteren Bereich des Reinigungsraums 2 angeordnet, wodurch der untere Wascharm 5 mit Reinigungsfluid 100 speisbar ist. Das Reinigungsfluid 100 kann vom Sumpf 11 mittels Umwälzpumpe 19 in der Umwälzleitung 20 zu den zwei Reinigungsfluidzuläufe 3 leitbar sein. Von den Reinigungsfluidzuläufen 3 kann das Reinigungsfluid 100 über die beiden Wascharme 5 im Reinigungsraum 2 verteilbar sein, wodurch die Reinigungsgüter und/oder der Reinigungsraum reinigbar und/oder desinfizierbar sind.

Der Reinigungsfluideingang 102 ist mit der Umwälzleitung 20 verbunden. Dadurch kann die Reinigungsanlage 1 mit frischem Reinigungsfluid 100, insbesondere mit Wasser, versorgbar sein.

Die Reinigungsanlage 1 umfasst zudem einen Plasmareaktor 4, durch welchen Wasser mit Plasma aktivierbar ist. Der Plasmareaktor 4 umfasst einen Gaseingang 101, durch welchen komprimierte Luft in den Plasmareaktor 4 geleitet werden kann. Im Plasmareaktor 4 kann die komprimierte Luft zu Plasma gewandelt werden. Der Plasmareaktor 4 ist durch eine Plasmaleitung 13 mit der Umwälzleitung 20 verbunden. Das erzeugte Plasma kann durch die Plasmaleitung 13 in die Umwälzleitung 20 geleitet werden. Dadurch kann das Plasma mit dem Reinigungsfluid 100, insbesondere Wasser, vermischt werden, wodurch plasmaaktiviertes Wasser herstellbar ist. Die Reinigungsanlage 1 umfasst zudem eine Plasmaverteilungsvorrichtung 9, welche an der Verbindungsstelle zwischen Plasmaleitung 13 und Umwälzleitung 20 angeordnet ist. Durch die Plasmaverteilungsvorrichtung 9 kann das Plasma im Reinigungsfluid 100 dispergiert werden.

Das Reinigungsfluid 100 kann über eine Abflussleitung (nicht dargestellt) verworfen werden.

**Figur 2** zeigt eine schematische Darstellung einer Reinigungsanlage 1 mit Zugabe des plasmaaktivierten Wassers im Sumpf 11 des Reinigungsraumes 2.

Die Reinigungsanlage 1 unterscheidet sich von jener, welche in Fig. 1 dargestellt ist, durch die Anordnung der Plasmaleitung 13 und der Plasmaverteilungsvorrichtung 9. In Ausführungsform, welche in Fig. 2 dargestellt ist, verbindet die Plasmaleitung 13 den Plasmareaktor 4 mit dem Sumpf 11 des Reinigungsraums 2. Dadurch kann Plasma in den, mit Reinigungsfluid 100 gefüllten, Sumpf 11 leitbar sein. Das Reinigungsfluid 100, insbesondere Wasser, kann im Sumpf 11 mit Plasma aktiviert werden und anschliessend vom Sumpf 11 mittels Umwälzpumpe 19 in der Umwälzleitung 20 zu den zwei Reinigungsfluidzuläufe 3 leitbar sein.

Die Plasmaverteilungsvorrichtung 9 ist an der Verbindungsstelle zwischen Plasmaleitung 13 und Sumpf 11 angeordnet.

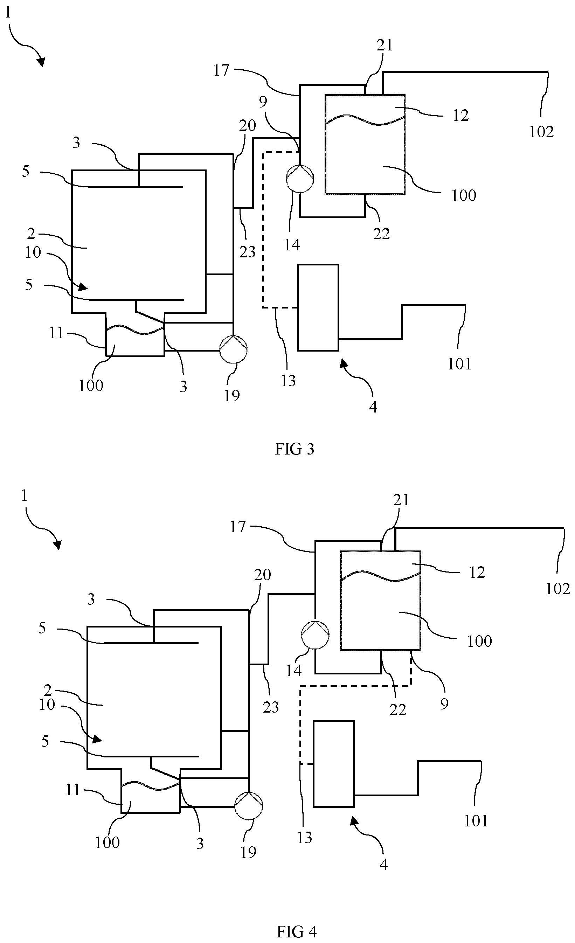

**Figur 3** zeigt eine schematische Darstellung einer Reinigungsanlage 1 mit Zugabe des plasmaaktivierten Wassers in der Zirkulationsleitung 17 der Aufbereitungsanlage 12.

Die Reinigungsanlage 1 unterscheidet sich von jener, welche in Fig. 1 dargestellt ist, durch die Anordnung einer Aufbereitungsanlage 12 zwischen dem Reinigungsfluideingang 102 und der Umwälzleitung 20. Zudem unterscheidet sich die Reinigungsanlage 1 sich von jener, welche in Fig. 1 dargestellt ist, durch die Anordnung der Plasmaleitung 13 und der Plasmaverteilungsvorrichtung 9.

Die Aufbereitungsanlage 12 umfasst einen Reinigungsfluidzulauf 21 und einen Reinigungsfluidablauf 22. Zudem ist der Reinigungsfluideingang 102 mit der Aufbereitungsanlage 12 verbunden. Durch den Reinigungsfluideingang 102 kann frisches Reinigungsfluid 100 in die Aufbereitungsanlage 12 geleitet werden. Durch den Reinigungsfluidzulauf 21 kann verwendetes und/oder zirkulierendes Reinigungsfluid 100 in die Aufbereitungsanlage 12 geleitet werden. Sowohl der Reinigungsfluideingang 102 als auch der Reinigungsfluidzulauf 21 sind im oberen Bereich der Aufbereitungsanlage 12 angeordnet. Im unteren Bereich ist der Reinigungsfluidablauf 22 angeordnet, durch welchen Reinigungsfluid 100 aus der Aufbereitungsanlage 12 geleitet werden kann.

Die Aufbereitungsanlage 12 umfasst eine Umwälzpumpe 14 und eine Umwälzleitung 17. Reinigungsfluid 100 kann vom Reinigungsfluidablauf 22 mittels Umwälzpumpe 14 in der Umwälzleitung 17 zum Reinigungsfluidzulauf 21 leitbar sein. Dadurch kann das Reinigungsfluid 100 in der Aufbereitungsanlage 12 umwälzbar sein und stehendes Reinigungsfluid 100 vermeidbar sein. Des Weiteren kann mittels Umwälzpumpe 14 Reinigungsfluid 100 vom Reinigungsfluidablauf 22 über die Verbindungsleitung 23 in die Umwälzleitung 20 des Reinigungsraums 2 leitbar sein. Dadurch kann Reinigungsfluid 100 zu den zwei Reinigungsfluidzuläufe 3 leitbar sein.

Das Reinigungsfluid 100 kann, wie zu Fig. 1 beschreiben, vom Sumpf 11 mittels Umwälzpumpe 19 in der Umwälzleitung 20 zu den zwei Reinigungsfluidzuläufe 3 leitbar sein. Des Weiteren kann das Reinigungsfluid 100, zur Aufbereitung, vom Sumpf 11 mittels Umwälzpumpe 19 über die Umwälzleitung 20 und Verbindungsleitung 23 in die Umwälzleitung 17 leitbar sein. Von der Umwälzleitung 17 kann das Reinigungsfluid 100 in die Aufbereitungsanlage 12 leitbar sein.

Der Plasmareaktor 4 ist durch die Plasmaleitung 13 mit der Umwälzleitung 17 verbunden. Das erzeugte Plasma kann durch die Plasmaleitung 13 in die Umwälzleitung 17 geleitet werden. Dadurch kann das Plasma mit dem Reinigungsfluid 100, insbesondere Wasser, vermischt werden, wodurch plasmaaktiviertes Wasser herstellbar ist.

**Figur 4** zeigt eine schematische Darstellung einer Reinigungsanlage 1 mit Zugabe des plasmaaktivierten Wassers in der Aufbereitungsanlage 12.

Die Reinigungsanlage 1 unterscheidet sich von jener, welche in Fig. 3 dargestellt ist, durch die Anordnung der Plasmaleitung 13 und der Plasmaverteilungsvorrichtung 9. In der in Fig. 4 dargestellten Ausführungsform verbindet die Plasmaleitung 13 den Plasmareaktor 4 mit der Aufbereitungsanlage 12. Dadurch kann Plasma in die mit Reinigungsfluid 100 gefüllte der Aufbereitungsanlage 12 leitbar sein. Das Reinigungsfluid 100, insbesondere Wasser, kann in der Aufbereitungsanlage 12 mit Plasma aktiviert werden und anschliessend mittels Umwälzpumpe 14 vom Reinigungsfluidablauf 22 über die Verbindungsleitung 23 in die Umwälzleitung 20 des Reinigungsraums 2 leitbar sein. Dadurch kann Reinigungsfluid 100 zu den zwei Reinigungsfluidzuläufe 3 leitbar sein. Die Plasmaverteilungsvorrichtung 9 ist an der Verbindungsstelle zwischen Plasmaleitung 13 und der Aufbereitungsanlage 12 angeordnet.

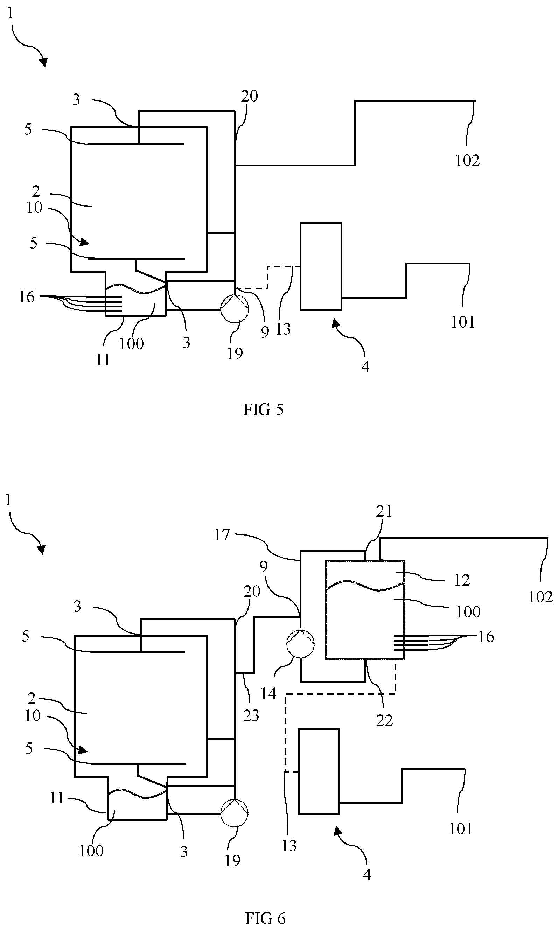

**Figur 5** zeigt eine schematische Darstellung der in Fig. 1 dargestellten Reinigungsanlage 1 mit einer Qualitätsüberwachungsvorrichtung 16 im Reinigungsraum 2.

Die Qualitätsüberwachungsvorrichtung 16 ist im Sumpf 11 des Reinigungsraums 2 angeordnet und umfasst vier Sensoren. Die vier Sensoren sind als pH-Sensor, Temperatursensor, Leitfähigkeitssensor und TOC-Sensor ausgebildet. Die Qualitätsüberwachungsvorrichtung 16 ermöglicht die Überwachung der keimreduzierenden Wirkung des Reinigungsfluids 100, insbesondere des plasmaaktivierten Wassers. Durch die Qualitätsüberwachungsvorrichtung 16 kann der Plasmareaktor 4 und/oder die Umwälzpumpe 19 regelbar sein.

**Figur 6** zeigt eine schematische Darstellung der in Fig. 4 dargestellten Reinigungsanlage 1 mit einer Qualitätsüberwachungsvorrichtung 16 in der Aufbereitungsanlage 12.

Die Qualitätsüberwachungsvorrichtung 16 ist in der Aufbereitungsanlage 12 angeordnet und umfasst vier Sensoren. Die vier Sensoren sind als pH-Sensor, Temperatursensor, Leitfähigkeitssensor und TOC-Sensor ausgebildet. Die Qualitätsüberwachungsvorrichtung 16 ermöglicht die Überwachung der keimreduzierenden Wirkung des Reinigungsfluids 100, insbesondere des plasmaaktivierten Wassers. Durch die Qualitätsüberwachungsvorrichtung 16 kann der Plasmareaktor 4 und/oder die Umwälzpumpe 19 und/oder die Umwälzpumpe 14 regelbar sein.

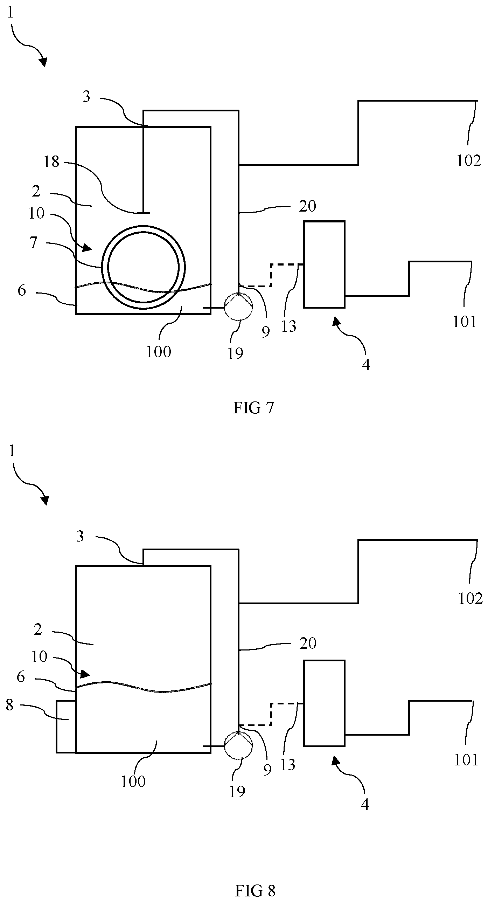

**Figur 7** zeigt eine schematische Darstellung einer Reinigungsanlage 1 mit einer Reinigungstrommel 7.

Die Reinigungsanlage 1 unterscheidet sich von jener, welche in Fig. 1 dargestellt ist, durch die Reinigungseinrichtung 10. Die Reinigungseinrichtung 10 umfasst ein Reinigungsbecken 6, eine Reinigungstrommel 7 und eine Sprühdüse 18. Das Reinigungsbecken 6 ist zumindest teilweise mit Reinigungsfluid 100 befüllt. Die Reinigungstrommel 7 kann Reinigungsgüter aufnehmen und ist derart angeordnet, dass sie zumindest teilweise durch das Reinigungsfluid 100 benetzbar ist. Die Sprühdüse 18 ist mit dem Reinigungsfluidzulauf 3 verbunden und oberhalb der Reinigungstrommel 7 angeordnet. Durch die Rotation der Reinigungstrommel 7 können die Reinigungsgüter durch die Reinigungsflüssigkeit 100 bewegbar sein, wodurch diese gereinigt und/oder desinfiziert werden können.

Durch die Sprühdüse 18 kann das umgewälzte Reinigungsfluid 100 in den Reinigungsraum 2 geleitet werden. Zudem kann dadurch die Reinigung und/oder Desinfektion der Reinigungsgüter verbesserbar sein.

**Figur 8** zeigt eine schematische Darstellung einer Reinigungsanlage 1 mit einem Ultraschallgenerator 8.

Die Reinigungsanlage 1 unterscheidet sich von jener, welche in Fig. 1 dargestellt ist, durch die Reinigungseinrichtung 10. Die Reinigungseinrichtung 10 umfasst ein Reinigungsbecken 6 und einen Ultraschallgenerator 8. Das Reinigungsbecken 6 ist zumindest teilweise mit Reinigungsfluid 100 befüllt.

Die Reinigungsgüter können vollständig von Reinigungsfluid 100 bedeckbar sein. Durch den Ultraschallgenerator 8, welcher am Reinigungsbecken 6 angeordnet ist, kann Ultraschall erzeugt werden, wodurch die Reinigungsleistung und/oder Desinfektionsleistung verbessert werden kann.

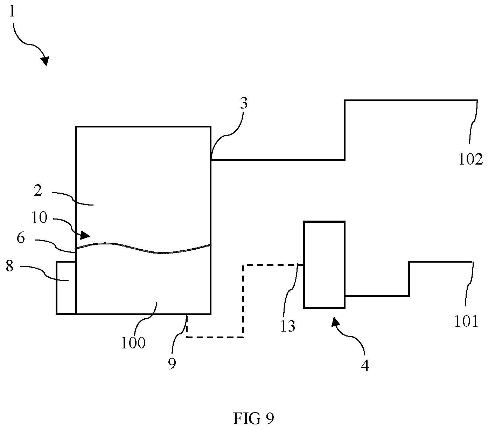

**Figur 9** zeigt eine schematische Darstellung einer Reinigungsanlage 1 mit einem Ultraschallgenerator 8.

Die Reinigungsanlage 1 umfasst einen Reinigungsfluidzulauf 3 und einen Reinigungsraum 2, welcher eine Reinigungseinrichtung 10 umfasst. Der Reinigungsfluideingang 102 ist mit dem Reinigungsfluidzulauf 3 verbunden, welcher im oberen Bereich des Reinigungsraums 2 angeordnet, wodurch das Reinigungsbecken 6 mit frischem Reinigungsfluid 100, insbesondere Wasser, speisbar ist.

Die Reinigungseinrichtung 10 umfasst ein Reinigungsbecken 6 und einen Ultraschallgenerator 8. Das Reinigungsbecken 6 ist zumindest teilweise mit Reinigungsfluid 100 befüllt. Die Reinigungsgüter können vollständig von Reinigungsfluid 100 bedeckbar sein. Durch den Ultraschallgenerator 8, welcher am Reinigungsbecken 6 angeordnet ist, kann Ultraschall erzeugt werden, wodurch die Reinigungsleistung und/oder Desinfektionsleistung verbessert werden kann.

Die Reinigungsanlage 1 umfasst zudem einen Plasmareaktor 4, durch welchen Wasser mit Plasma aktivierbar ist. Der Plasmareaktor 4 umfasst einen Gaseingang 101, durch welchen komprimierte Luft in den Plasmareaktor 4 geleitet werden kann. Im Plasmareaktor 4 kann die komprimierte Luft zu Plasma gewandelt werden. Der Plasmareaktor 4 ist durch eine Plasmaleitung 13 mit dem Reinigungsbecken 6 verbunden. Das erzeugte Plasma kann durch die Plasmaleitung 13 in das Reinigungsbecken 6 geleitet werden. Dadurch kann das Plasma mit dem Reinigungsfluid 100, insbesondere Wasser, vermischt werden, wodurch plasmaaktiviertes Wasser herstellbar ist. Die Reinigungsanlage 1 umfasst zudem eine Plasmaverteilungsvorrichtung 9, welche an der Verbindungsstelle zwischen Plasmaleitung 13 und Reinigungsbecken 6 angeordnet ist. Durch die Plasmaverteilungsvorrichtung 9 kann das Plasma im Reinigungsfluid 100 dispergiert werden.

Das Reinigungsfluid 100 kann über eine Abflussleitung (nicht dargestellt) verworfen werden.

## Patentansprüche

1. Reinigungsanlage (1), insbesondere Sterilisations- und/oder Desinfektionsanlage, zur Reinigung von medizinischen oder pharmazeutischen Instrumenten oder Gütern, umfassend einen Reinigungsraum (2) mit einer Reinigungseinrichtung (10), in dem die Güter reinigbar sind, einen Reinigungsfluidzulauf (3) zur Zuführung von Reinigungsfluid (100), **dadurch gekennzeichnet, dass** ein Plasmareaktor (4) ausgebildet ist, durch den Wasser mit Plasma aktivierbar ist und Plasma im Plasmareaktor (4) mittels Gas, vorzugsweise mittels Luft und/oder Stickstoff, herstellbar ist und das plasmaaktivierte Wasser als Reinigungsfluid (100) verwendbar ist.

2. Reinigungsanlage (1) nach Anspruch 1, **dadurch gekennzeichnet, dass** die Reinigungseinrichtung (10) zumindest einen beweglichen Wascharm (5) umfasst.

3. Reinigungsanlage (1) nach Anspruch 1, **dadurch gekennzeichnet, dass** die Reinigungseinrichtung (10) ein Reinigungsbecken (6) und eine Reinigungstrommel (7) und vorzugsweise mindestens eine Sprühdüse (18) umfasst, wobei die Reinigungstrommel (7) zumindest teilweise im Reinigungsbecken (6) angeordnet ist.

4. Reinigungsanlage (1) nach einem der vorhergehenden Ansprüche, **dadurch gekennzeichnet, dass** die Reinigungseinrichtung (10) ein Reinigungsbecken (6) und einen Ultraschallgenerator (8) umfasst.

5. Reinigungsanlage (1) nach einem der vorhergehenden Ansprüche, **dadurch gekennzeichnet, dass** die Reinigungsanlage (1) eine Plasmaleitung (13) und eine Plasmaverteilungsvorrichtung (9), insbesondere eine Plasmadispergiervorrichtung, umfasst.

6. Reinigungsanlage (1) nach einem der vorhergehenden Ansprüche, **dadurch gekennzeichnet, dass** der Plasmareaktor (4) über die Plasmaleitung (13) direkt oder indirekt mit dem Reinigungsraum (2) verbunden ist, insbesondere mit dem Sumpf (11) eines Reinigungsraumes (2).

7. Reinigungsanlage (1) nach einem der vorhergehenden Ansprüche, **dadurch gekennzeichnet, dass** eine Aufbereitungsanlage (12) ausgebildet ist, in der das Reinigungsfluid (100) aufbereitbar ist.

8. Reinigungsanlage nach Anspruch 7, **dadurch gekennzeichnet, dass** die Plasmaleitung (13) vom Plasmareaktor (4) zur Aufbereitungsanlage (12) ausgebildet ist und der Reinigungsfluidzulauf (3) von der Aufbereitungsanlage (12) zum Reinigungsraum (2) verläuft.

9. Reinigungsanlage (1) nach einem der Ansprüche 7 oder 8, **dadurch gekennzeichnet, dass** die Aufbereitungsanlage (12) eine Umlaufleitung (17) mit einer Umlaufpumpe (14) umfasst.

10. Reinigungsanlage (1) nach einem der vorhergehenden Ansprüche, **dadurch gekennzeichnet, dass** eine Qualitätsüberwachungsvorrichtung (16) zur Überwachung des plasmaktivierten Wassers ausgebildet ist.

11. Reinigungsanlage (1) nach Anspruch 10, **dadurch gekennzeichnet, dass** die Qualitätsüberwachungsvorrichtung (16) einen PH-Sensor und/oder einen Leitfähigkeitssensor und/oder einen Temperatursensor und/oder einen ORP (oxidation-reduction-potential)-Sensor und/oder einen TOC (total-organiccarbon)-Sensor umfasst.

12. Verfahren zur Reinigung, insbesondere Desinfektion oder Sterilisierung von Gütern, insbesondere medizinischen und/oder pharmazeutischen Gütern, in einer Reinigungsanlage (1) nach einem der vorhergehenden Ansprüche, wobei zunächst aus Wasser mit Plasma durch Vermischen plasmaaktiviertes Wasser erstellt wird und das plasmaaktivierte Wasser, insbesondere das plasmaaktive Wasser und ein zusätzliches Reinigungsmittel, zum Reinigen von Gütern in einem Reinigungsraum (2) verwendet wird.

13. Verfahren nach Anspruch 12, **dadurch gekennzeichnet, dass** das plasmaaktivierte Wasser in einer Aufbereitungsanlage (12) gesammelt wird.

14. Verfahren nach Anspruch 12, **dadurch gekennzeichnet, dass** das plasmaaktivierte Wasser in den Reinigungsraum (2) geleitet wird.

15. Verfahren nach einem der vorhergehenden Ansprüche 12 bis 14, **dadurch gekennzeichnet, dass** das plasmaaktivierte Wasser nach der Herstellung innerhalb von maximal 2 Stunden, insbesondere 1 Stunde zum Reinigen im Reinigungsraum (2) verwendet wird.

16. Verfahren nach einem der vorhergehenden Ansprüche 12 bis 15, **dadurch gekennzeichnet, dass** das plasmaaktivierte Wasser mit einer Qualitätsüberwachung (16) überwacht wird.
